# EUROPEAN PATENT APPLICATION

(11) **EP 2 987 481 A1**
(43) Date of publication of application: **24.02.2016**
(21) Application number: 15180533.0
(22) Date of filing: 11.08.2015
(51) Int. Cl.: A61K 8/06, A61K 8/73, A61Q 5/06

(54) **THICKENER FOR SEMI-PERMANENT HAIR DYES**

(30) Priority: 18.08.2014 GB 201414586
(71) Applicant: Rheolab Ltd, Leeds, West Yorkshire LS19 7LX (GB)
(72) Inventor: WAKELIN, Alan, Leeds, West Yorkshire LS19 7LX (GB); FRIEND, Rachel, Leeds, West Yorkshire LS19 7LX (GB)
(74) Representative: Cookson, Barbara Elizabeth

(57) **Abstract**

An independently stable thickening composition suitable for use with cationic and nonionic hair dyes comprises only 3 components: an inverse emulsion in which finely divided HEC has been dispersed together with a stabilising component to prevent sedimentation. Since cationic dyes used in semi-permanent hair dyes thickened with such a composition are unable or less able to bond with the HEC in the thickener, the product requires less dye to produce the same intensity of colour as less dye is wasted.

## Description

### Technical Field

The present invention relates to the thickening of semi-permanent hair dyes for use in the cosmetics industry. The invention also relates to compositions for liquid and gel semi-permanent hair dyes particularly using cationic dyes.

### Background Art

Semi-permanent hair dyes are the most common method of temporarily changing hair colour. They are routinely sold through supermarkets in tubes for application at home by comb or brush. They are typically left on the hair for 30 minutes. Therefore, they are required to be thick enough, that is sufficiently viscous, to remain on the hair for this period. At present the only suitable thickeners are natural thickeners such as hydroxyl ethyl cellulose (HEC) or its derivatives. Synthetic thickeners, such as acrylates have been found to be ineffective in thickening semi-permanent hair dyes.

When a HEC thickener is used, it is removed when the hair is rinsed after dyeing. Because the dye becomes attached to the HEC, there is a significant waste of dye when the hair is rinsed. This results in a loss of colour intensity.

This loss of dye also results in the discharge of coloured waste water that requires treatment.

When manufacturing a range of semi-permanent dyes, it is necessary to vary the amount of thickener added to the formulation depending on the type and colour of hair dye being used to take account of the anticipated waste and the variable attraction of the dies to HEC. This is a significant complication in the manufacture of semi-permanent hair dyes.

This technical problem of excessive dye use in semi-permanent hair dyes is solved by the stable thickening composition of the present invention as defined in claim 1.

This inventive composition is limited to three components and comprises only an inverse emulsion in which finely divided hydroxyl ethyl cellulose (HEC) has been dispersed together with a stabilising component to prevent sedimentation. The thickening composition is stable and can be stored until required for use without the components coming out of suspension.

US 2013/0189206 A1 (GOGET et al) 25 July 2103 describes a 2 composition hair dye comprising a water in oil inverse emulsion containing the hair dye and a separate composition which is an oxidising agent. Goget addresses the field of permanent or oxidation dyes which is distinct from the technical field of semi-permanent dyeing which is the field of the present invention. Accordingly, the emulsion composition is required to contain a lipophilic surfactant and a base, neither of which is required in a semi-permanent dye. The emulsion composition taught by Goget suggests a multitude of potential thickening agents.

WO 2013/004784 A2 (L'Oreal) 10 Jan 2103 is again concerned with the technical field of permanent oxidation dyes using two compositions. It is taught that additional cationic dyes might be added and that some compositions may contain HEC or a multitude of other organic thickeners. No independently stable thickening composition suitable for use only with cationic dyes is contemplated by this disclosure.

### Advantages of the invention

Since cationic dyes used in semi-permanent hair dyes thickened with a composition in accordance with the invention are unable or less able to become attached to the HEC in the thickener, the product requires less dye to produce the same intensity of colour as dye is not wasted. Alternatively, a more intense dyeing effect can be produced with the same amount of dye.

The thickening compositions claimed provide the required viscosity to allow application, but avoid the loss of dye in wash off.

An inverse emulsion is an emulsion containing both an oil-in-water emulsifier and water-in-oil emulsifier, wherein the aqueous phase is dispersed in the organic phase in very small drops. Cosmetic inverse emulsions are commercially available. They are used as thickeners for cosmetics. See for example US 2008051492 A (LAMBERTI SPA) 28 Feb 2008 Inverse emulsions are commonly used as liquid thickeners of aqueous systems in cosmetic and other applications. These inverse emulsions typically consist of an oil continuous phase with a water soluble polymer dissolved in aqueous droplets.

HEC is a hydrophilic thickener that does not dissolve in oil but does dissolve in water to give a clear solution. It and its derivatives are widely available in the cosmetic supply industry and are supplied in various grades. It is a natural powder thickener.

### Description of embodiments

A manufacturing process for creating embodiments of the thickening composition of the invention will now be described, by way of example only.

A thickener extracted from a natural source, preferably finely milled grade of HEC of particle size between 0.5 and 1.80 mm, is prepared as a dry powder.

The inverse emulsion used is an oil continuous phase with a water soluble polymer dissolved in aqueous droplets. The aqueous droplets are between 1 and 5 microns, surrounded by a surfactant capable of phase inversion when surplus water is added to the liquid thickener.

A third component is required to prevent irreversible sedimentation of the natural powder HEC from the inverse emulsion. The third component acts as a stabiliser of the oil continuous phase. This third component can be an oil thickener in the form of a hydrophobic polymer such as an acrylic copolymer added at a level of between 0.5wt% and 1.5 wt% of the finished product.

These three components are combined into a finished liquid thickener by the following method-
- First, between 65 and 75 grams of liquid inverse emulsion are agitated using a high shear mixer rotating at between 500 and 1000 rpm to create a vortex.
- 30 grams of the powder grade natural HEC thickener of the above specification are added into the vortex of the stirring inverse emulsion over a period of 30-45mins. (Faster addition causes aggregations of powder thickener to form which do not subsequently disperse).
- Then between 0.5 grams and 1.5 grams of a stabilising component (hydrophobic polymer) is added over a period of around 20 minutes.
- The mixture is stirred for a further 30 minutes.

The resulting combined thickener system might be expected to separate by sedimentation, during the period of storage for use, in a fashion that would not be capable of re-suspension. However, it is found that by the selection of the three components described in accordance with the invention, a product that is stable for a period of up to a year can be produced.

This combined thickener can be made using different proprietary HEC powders such as NATURSOL® HDV or C100 as well as the basic chemical.

This blended or combined thickener is then employed in the manufacture of semi-permanent hair dyes, using less hair dye than would be expected for the desired colour shade measured either visually or by intensity measurement.

An example of a semi- permanent hair dye formulation in which this combined thickener is used is as follows.

### Example 1

| | |
|---|---|
| Aqua | 80 pts |
| Citric Acid | 1.0 pts |
| Cocamidopropyly Betaine | 1.5 pts |
| Cocamidopropylamine oxide | 1.0 pts |
| Polyquaternium 7 | 2.0 pts |
| Monoethanolamine | 1.0 pts |
| Combined thickener incorporating | |
| HEC *or* | 1.0 pts |
| NATURSOL® HDV or C100 | 2.25 pts |
| Dye -(JAROCOL® Violet 1.4D) | 2.0 pts |

This is a cationic system. The formulation was found to be satisfactory with other commercial cationic dyes and direct dyes such as JAROCOL NHEAP (red) and direct blue, brown, black, yellow and red dyes as well as non- ionic dyes. The formulation is unsuitable for anionic dyes.

The colour yield enhancements which are claimed were measured using the following method

Measurement were taken using a DataColor ® Spectraflash 600® spectrophotometer. Colours were tested to method CMC 2:1 which is a Colour Equation standard commonly used in the textile industry.

The example hair dye formulations were made up with conventional HEC and with the same amount of the combined hair dye thickener of the present invention.

The resulting colour yield differences when the combined hair dye thickener were compared to formulations containing the same amount of dye and conventional HEC thickener were as follows:

### Colour Intensity Results

| | |
|---|---|
| JAROCOL® Violet 1.4D | +38% |
| JAROCOL® NHEAP (red) | +50% |
| JAROCOL® Red 3 using NATURSOL® HDV in the combined thickener | +80% |

Therefore, it is possible to use less dye in a semi-permanent hair dye formulation using the combined thickener of the invention and achieve the same colour yield. Alternatively, a deeper shade of colour can be produced with the same amount of dye. Accordingly, the manufacturer has greater control over ingredients and shades offered. In some cases the hair colour may be limited due to the dyestuff solubility and therefore the ability to produce an increase shade with less dye is highly desirable.

It is also found that the use of this combined thickener allows the use of a single w/v concentration of thickener to dyestuff in manufacture of semi-permanent hair dyes across a wide range of types and colours of hair dyes. This is because the use of the combined thickener eliminates the variability due to interaction of the specific dye with the HEC.

The following terms are registered trade marks (indicated by the ® symbol) and should be read as such wherever they occur in this document: NATURSOL, JAROCOL and DATACOLOR.

## Claims

1. A stable thickening composition suitable for use with cationic or non-ionic hair dyes which comprises only an inverse emulsion in which finely divided hydroxyl ethyl cellulose (HEC) has been dispersed together with a stabilising component to prevent sedimentation.

2. A composition as claimed in claim 1, wherein the stabilising component is a hydrophobic polymer.

3. A composition as claimed in claim 1, wherein the stabilising component is an acrylic copolymer.

4. A composition as claimed in any one of the preceding claims, wherein the HEC is combined in the inverse emulsion in the proportions of 30 g HEC to 65-75 grams of liquid inverse emulsion.

5. A composition as claimed in any one of the preceding claims, wherein the stabilising component is present at 0.5 to 1.5wt%.

6. A semi-permanent hair dye liquid or gel composition comprising a cationic or non-ionic dye together with a thickening composition as claimed in any one of the preceding claims.

7. A method of making a thickening composition as claimed in any one of claims 1 to 5, comprising the steps of mixing the liquid inverse emulsion in a high shear mixer in order to create a vortex and introducing the HEC powder over a period of 30 to 45 minutes, and then adding the stabilising component.
